# FASCICULE DE BREVET EUROPEEN

(11) **EP 2 416 846 B1**
(45) Date de publication et mention de la délivrance du brevet: **26.12.2018**
(21) Numéro de dépôt: 10714904.9
(22) Date de dépôt: 31.03.2010
(51) Int. Cl.: A61N 1/30, A61N 5/06, A61N 1/32

(54) **PROCEDE ET APPAREIL DE SOIN COSMETIQUE DE LA PEAU**
VERFAHREN UND GERÄT ZUR KOSMETISCHEN HAUTPFLEGE
METHOD AND APPLIANCE FOR COSMETIC SKINCARE

(30) Priorité: 31.03.2009 FR 0901584
(43) Date de publication de la demande: 15.02.2012
(73) Titulaire: Leonardi Kader, Simone Nadia, 06000 Nice (FR)
(72) Inventeur: Leonardi Kader, Simone Nadia, 06000 Nice (FR)
(74) Mandataire: Cornuejols, Georges
(86) Numéro de dépôt international: PCT/FR2010/000274
(87) Numéro de publication internationale: WO 2010/112708

(56) Documents cités:
- CA-A1- 2 363 383
- KR-A- 20030 016 533
- US-A1- 2005 177 207
- US-A1- 2006 253 078
- US-A1- 2007 060 862

## Description

La présente invention a pour objet un appareil de soin de la peau, à visée esthétique notamment, qui est destiné à être utilisé en cosmétologie, par exemple pour traiter les taches pigmentaires qui nuisent à l'aspect esthétique de la peau, pour réduire certains effets visibles du vieillissement, notamment pour un rajeunissement de la peau, ou pour une augmentation de la fermeté de la peau.

Elle a aussi pour objet un procédé de soin cosmétologique, notamment esthétique, dont la mise en oeuvre s'effectue au moyen de l'appareil en question. Ce procédé est tout particulièrement utile pour soumettre la peau à des actions d'ordre physique en complément à des principes actifs connus pour présenter une activité plutôt d'ordre chimique, voire biochimique, comme ceux que l'on applique couramment sur la peau sous forme de crèmes cosmétiques, de gels, de solutions, ou toute forme convenant à une application locale. Ceci toutefois n'exclut pas que l'appareil suivant l'invention puisse trouver également utilité pour aider à l'efficacité d'autres produits à appliquer sur la peau, y compris en assistance à des traitements par des principes actifs.

Les tâches pigmentaires cutanées, telles que chloasma, mélasma, tâches de vieillesse, peuvent apparaître sur le visage, le cou, le décolleté, les avant-bras, le dessus des mains, les zones de frottement corporel. Elles ne sont pas l'apanage des personnes âgées, car on en déplore également chez des personnes relativement jeunes. D'une manière générale, outre le vieillissement naturel de la peau, elles peuvent avoir des origines aussi diverses que le soleil, la grossesse, certains traitements médicaux, la contraception, pour ne citer que quelques exemples.

Pour réduire la visibilité de ces taches, voire pour les faire disparaître, il existe des techniques qui consistent à éliminer la couche cornée de la peau responsable de la présence des taches. Soit on procède en enlevant la partie supérieure de l'épiderme à l'endroit du siège de la tâche, par dermabrasion ou peeling, soit on cherche à brûler les pigments fautifs, par exposition à un rayonnement tel qu'un faisceau laser, ce qui conduit à brûler la peau à l'endroit des taches. Ces traitements, qui sont agressifs pour l'épiderme, ont en outre l'inconvénient de ne pas convenir à tous les types de peaux. Par exemple, l'exposition à un rayonnement laser est notamment déconseillée pour les peaux mates (en particulier, on observe dans certains cas une hyperpigmentation d'origine réactionnelle). Il s'ensuit que la mise en oeuvre de ces techniques demande la pratique de spécialistes, capables de définir des adaptations spécifiques au cas par cas, suivant le type de peau du patient (notamment entre les peaux claires et les peaux mates), suivant la localisation des taches à traiter, suivant leur origine ou leur ancienneté, suivant encore l'état général de la peau au moment du traitement.

A ces traitements à action trop physiquement agressive on préfère donc des techniques de soin cosmétologique, passant ou non par une action d'ordre chimique, plus spécialement ceux qui impliquent l'application de courants électriques. On connaît ainsi des techniques qui utilisent des courants pulsés pour provoquer des phénomènes d'électroporation, d'autres qui utilisent des courants continus de polarité constante dans des conditions propres à provoquer des phénomènes d'électrophorèse dans l'épiderme.

A titre l'exemple, le document US2007/060862 décrit un appareil pour le soin de la peau, comprenant une membrane et des moyens de production d'un courant électrique pulsé ou continu, et des moyens d'émission de lumière, notamment des LED. Les LED modulent le courant qui traverse la membrane pour former un courant direct pulsatile, afin d'améliorer la délivrance des médicaments, stimuler les réponses biologiques. A cette occasion l'appareil peut comprendre deux LED émettant à des longueurs d'ondes de 400-500 nm et 700-1000 nm. Cet appareil possède une efficacité limitée.

Le document KR 2003 0016533 décrit un appareil de thermothérapie de taille compacte utilisant l'infrarouge. Cet appareil comprend un moyen d'émission d'un rayonnement infrarouge, des moyens d'émission de chaleur et des moyens d'émission d'un courant. Ici encore, cet appareil possède une efficacité limitée.

Le document US 2006/253078 décrit quant à lui un appareil de traitement de la peau comprenant notamment une membrane destinée à être en contact avec la peau, une source d'énergie, deux électrodes conductrices, un réservoir ; la source d'énergie est en communication électrique avec la première électrode et la deuxième électrode, les électrodes étant disposées de manière à être en communication ionique avec un transporteur présent dans le réservoir. L'appareil peut être conçu de manière à comprendre une LED ; la lumière émise peut être en communication avec la membrane. La source d'énergie peut être un courant direct ou un courant pulsé. L'appareil peut, dans un mode de réalisation particulier, comprendre deux LED émettant à des longueurs d'ondes de 400-500 nm et 700-1000 nm. L'efficacité de cet appareil peut être améliorée.

Enfin, le document US 2005/177207 décrit un procédé pour inhiber la croissance cellulaire ou pour augmenter la mort cellulaire par électroporation d'un agent photosensible dans une cellule. Le procédé consiste plus particulièrement en l'administration d'un agent photosensible, permettant de traiter les désordres prolifératifs d'une cellule, par l'application d'une pulsion électrique à la cellule, d'une force et d'une durée suffisante pour permettre l'électroporation de la cellule avec l'agent photosensible, et l'application d'une lumière d'une longueur d'onde permettant l'activation de l'agent et traiter la maladie proliférative. La pulsion électrique peut être un champ électrique pulsé adapté via un appareil d'électroporation. La lumière peut être appliquée avant, pendant ou après l'électroporation. La longueur d'onde dépend de l'énergie nécessaire à l'activation de l'agent photosensible ; elle peut être de 560 nm.

Ces traitements sont en général mis en oeuvre en complément avec l'application de principes actifs contenus en mélange avec un véhicule approprié dans une composition pour application locale, crème, lotion, ou autre.

Pour une efficacité optimale et une agression minimale de la peau, l'invention propose de soumettre la peau à un cycle de plusieurs traitements différents qui ont chacun leur rôle propre sur la peau, dans des conditions où leurs effets respectifs se potentialisent mutuellement. Avantageusement, ces cycles de traitement ont une action synergique entre eux. Le procédé suivant l'invention comme l'appareil conçu pour sa mise en oeuvre ont la particularité de combiner l'application à la peau, en une succession prédéterminée, de séquences prédéfinies de courants continus et de courants pulsés associés à des séquences d'éclairement de la zone à traiter par des lumières de couleurs différentes.

Plus particulièrement, l'invention se traduit en un procédé qui consiste essentiellement à soumettre la peau à un cycle de séquences successives comportant au moins une première séquence de traitement de la peau par application d'un courant pulsé d'électroporation en combinaison avec l'exposition simultanée de la peau en cours de soin à de la lumière plus chaude, et une seconde séquence de traitement de la peau par application d'un courant continu d'électrophorèse en combinaison avec l'exposition simultanée de la peau en cours de soin à de la lumière de couleur moins chaude.

On entend par " plus chaude ", au sens de la présente invention, une couleur de longueur d'ondes comprise entre 692 nm et 800 nm, la borne inférieure de 692 nm n'étant pas comprise. Par exemple, la longueur d'ondes peut être comprise entre 764 nm et 780 nm. Il peut s'agir par exemple d'une couleur choisie parmi le rouge-orangé, le rouge et le rouge-violet. Avantageusement, l'application de cette couleur peut être apte à potentialiser l'effet d'électroporation. L'application de cette couleur peut augmenter l'effet de traitement de la peau dû à l'électroporation seule. Avantageusement, cette augmentation de l'effet de l'électroporation peut être d'au moins 2 fois, par rapport à la réalisation de l'électroporation seule. L'application de cette couleur peut avoir un effet synergique avec l'électroporation. La potentialisation de l'effet de l'électroporation peut être observée ou mesurée par tout moyen connu de l'homme du métier, par exemple par observation visuelle ou photographie numérique, cette liste n'étant pas limitative. Avantageusement, l'intensité lumineuse peut permettre de potentialiser les effets de l'application de la couleur. L'intensité lumineuse associée à cette couleur peut être comprise entre 70 et 150 candelas, par exemple entre 120 et 150 candelas, ou encore entre 130 et 150 candelas.

On entend par " moins chaude ", au sens de la présente invention, une couleur longueur d'ondes comprise entre 560 nm et 692nm, la borne inférieure de 560 nm n'étant pas comprise. Par exemple, la longueur d'ondes peut être comprise entre 580 nm et 692 nm, 692 nm étant inclus. Il peut s'agir par exemple d'une couleur choisie parmi le jaune, le jaune-orangé et l'orange. L'application de cette couleur peut augmenter l'effet de traitement de la peau dû à l'électrophorèse seule. Avantageusement, cette augmentation de l'effet de l'électroporation peut être d'au moins 2 fois par rapport à la réalisation de l'électrophorèse seule. L'application de cette couleur peut avoir un effet synergique avec l'électrophorèse. La potentialisation de l'effet de l'électrophorèse peut être observée ou mesurée par tout moyen connu de l'homme du métier, par exemple par observation visuelle ou photographie numérique, cette liste n'étant pas limitative. Avantageusement, l'intensité lumineuse peut permettre de potentialiser les effets de l'application de la couleur. Avantageusement, l'intensité lumineuse associée à cette couleur peut être comprise entre 30 et 69 candelas, par exemple entre 40 et 60 candelas. De préférence, la première séquence, dite d'électroporation, et la seconde séquence, dite d'électrophorèse, constituent deux étapes qui se succèdent dans cet ordre lors de la mise en oeuvre du procédé de l'invention. Quant aux couleurs de lumière à utiliser, il s'agit préférentiellement de lumière rouge pour l'étape d'électroporation et de lumière orange pour l'étape d'électrophorèse. On observe que dans les deux cas, la lumière utilisée reste dans la gamme des couleurs chaudes du spectre visible, celles donc qui correspondent à des longueurs d'onde relativement élevées.

Ainsi, l'invention se rapporte notamment à un procédé de soin esthétique de la peau, dans lequel la peau est soumise à un cycle de séquences successives comprenant, dans cet ordre, au moins une première séquence de soin de la peau, dite d'électroporation, comprenant l'application sur la peau d'un courant pulsé d'électroporation en combinaison avec l'exposition simultanée de la peau en cours de soin à de la lumière dont la longueur d'ondes est comprise entre 692 nm et 800 nm, et une seconde séquence de soin de la peau, dite d'électrophorèse, comportant l'application d'un courant continu d'électrophorèse en combinaison avec l'exposition simultanée de la peau en cours de soin à de la lumière de longueur d'ondes inférieure à celle appliquée lors de la première séquence de soin, par exemple comprise entre 560 nm à 692 nm.

On entend par " soin esthétique ", au sens de la présente invention, tout traitement non thérapeutique, visant à améliorer l'aspect de la peau saine. Il peut s'agir notamment de soin cosmétique, cosmétologique, cosméceutique. Le procédé peut par exemple permettre le traitement des tâches pigmentaires cutanées bénignes, non associées à une pathologie, telles que chloasma, mélasma, tâches de vieillesse, cette liste n'étant pas limitative. Avantageusement, le procédé peut permettre de réduire la visibilité de ces tâches. Il peut s'agir d'une réduction de la taille des tâches. Avantageusement, la taille des tâches peut être réduite d'un tiers, ou encore de moitié, voire de trois quarts. L'homme du métier peut observer la réduction de la taille de la tâche par toute méthode connue, par exemple par observation visuelle ou photographie numérique, cette liste n'étant pas limitative. Avantageusement, le soin peut permettre de rapprocher la teinte de la tâche pigmentaire de celle de la peau saine, par exemple de rendre la tâche plus claire. Par exemple, la tâche peut être rendue au moins deux fois, voire trois ou quatre fois plus claire. L'homme du métier peut observer le changement de teinte de la tâche par toute méthode connue, par exemple par observation visuelle ou photographie numérique, cette liste n'étant pas limitative. Avantageusement, le soin esthétique peut avoir comme but le rajeunissement global de la peau du visage, des mains et du corps, le traitement spécifique des rides, le relâchement cutané par augmentation de la fermeté, le traitement de la cellulite, le traitement des vergetures. Les peaux traitées peuvent être des peaux claires, les peaux mates et les peaux noires.

On entend par " patient ", au sens de la présente invention, tout individu sur lequel le procédé ou l'appareil est appliqué.

Par le recours à de la lumière se situant dans le spectre visible, de telle sorte qu'elle se définit par une notion de couleur, on peut considérer que l'invention fait application sur la peau de phénomènes de chromothérapie (on dit aussi chromathérapie), qu'elle propose de combiner de manière spécifique avec les traitements par application de courants électriques, en variant la couleur d'une étape à l'autre.

Dans les modes de mise en oeuvre préférés de l'invention, le même cycle de traitement comporte une troisième séquence consistant en un traitement de régénération de la peau par exposition à une lumière de couleur froide. On entend par " couleur froide ", au sens de la présente invention, une couleur longueur d'ondes comprise entre 330 nm et 560 nm, 560 nm étant compris. Il peut s'agir par exemple d'une couleur choisie parmi le bleu-violet, le bleu, le bleu-vert, le vert et le jaune-vert. Avantageusement, cette couleur peut être apte à améliorer la régénération des cellules de l'épiderme, par exemple à réhydrater les cellules de l'épiderme. Avantageusement, l'application de cette couleur peut permettre à la peau d'avoir un aspect plus lisse et/ou plus soyeux. Avantageusement, l'application de cette couleur peut permettre de raviver le teint. L'homme du métier peut observer ou mesurer une ou plusieurs de ces améliorations par toute méthode connue, par exemple par observation visuelle ou photographie numérique, cette liste n'étant pas limitative. Avantageusement, l'intensité lumineuse peut permettre de potentialiser les effets de l'application de la couleur. Avantageusement, l'intensité lumineuse associée à cette couleur peut être comprise entre 2 et 29 candelas, par exemple entre 10 et 20 candelas.

Cette couleur se situe typiquement dans la gamme des courtes longueurs d'onde, dont relève en particulier le bleu.

Conformément à des caractéristiques secondaires propres à l'invention, le cycle de traitement de la peau, en sa troisième séquence, consiste donc essentiellement en un traitement de régénération de la peau par chromothérapie, sous exposition à une lumière de couleur froide, telle que le bleu. Dans la plupart des applications, on trouvera intérêt à combiner ce traitement par chromothérapie avec un traitement par électrophorèse. Avantageusement, cette combinaison peut permettre de potentialiser l'effet de régénération de la peau. Durant la troisième étape du procédé, il est donc en général avantageux de poursuivre l'application d'un courant à action électrophorétique, comme celui de la seconde étape.

Ceci vaut notamment lorsque le courant est choisi de polarité adaptée pour contribuer par électrophorèse à la pénétration à travers l'épiderme de principes actifs contenus dans une composition pour application locale sur la peau. L'homme de l'art en la matière saura définir la polarité à utiliser, en général en fonction des propriétés du véhicule présent dans ladite composition en mélange avec les principes actifs. En effet, et comme on l'aura déjà compris de l'exposé de l'art antérieur, les conditions d'application préférées du procédé suivant l'invention prévoient que, préalablement au cycle d'application d'un courant et d'exposition simultanée à de la lumière colorée, en ses deux ou trois séquences, on dépose sur la peau une composition cosmétique pour application locale sur la peau. Dans le cadre de l'invention, une telle composition est de préférence sous forme de lotion, car cette présentation est favorable par sa fluidité à l'efficacité des traitements sous application d'un courant électrique, comparativement aux crèmes ou gels.

Avantageusement, un principe actif de la composition cosmétique déposée sur la peau peuvent avoir un effet anti-vieillissement, un effet hydratant, un effet de réduction de l'apparence de la cellulite, un effet de dépigmentation.

Par exemple, un principe actif peut être choisi parmi l'acide mandélique, l'acide salicylique, l'acide rétinoïque, le rétinol, la Forskoline, la carnitine, le glycérol, le sorbitol, l'acide hyaluronique, le silicone, la paraffine, cette liste n'étant pas limitative.

Un appareil selon l'invention, tel qu'il est conçu pour permettre de mettre en oeuvre le procédé ci-dessus comporte des moyens qui sont en soi classiques, du moins dans leur définition générale. Il en est ainsi principalement, d'une part, des moyens de production d'un courant électrique de polarité déterminée entre une électrode à amener au contact de la peau du patient dans la zone à traiter et une électrode opposée à porter à un potentiel de référence, que le patient tient en général dans la main pour cela. Il s'agit aussi, d'autre part, des moyens de production d'un rayonnement lumineux coloré, que l'on dirige vers la peau dans la zone à traiter, en général des lampes électriques, et plus particulièrement des diodes électroluminescentes. Les moyens de production d'un rayonnement lumineux coloré peuvent être ceux disponibles dans le commerce, comme par exemple des ampoules colorées.

Les moyens spécifiques de l'invention dans l'appareil en question concernent pour partie la commande automatique des moyens électriques et des moyens d'illumination, ainsi que la gestion automatisée du déroulement des séquences successives du cycle de traitement. Ils concernent aussi, d'autre part, la disposition des différents éléments fonctionnels d'action sur la peau dans leur montage mécanique dans une tête d'application à enveloppe isolante que le personnel soignant, professionnel praticien de la santé ou de l'esthétique, tient dans la main pour l'appliquer sur la peau du patient.

La commande automatique des moyens électriques et des moyens d'illumination peut être réalisée avec tout moyen disponible dans le commerce.

Dans son ensemble, l'appareil suivant l'invention comporte, au niveau de cette tête, une électrode à appliquer sur la zone de peau à traiter ainsi qu'un ensemble de diodes électroluminescentes en tant que moyens d'illumination disposés au voisinage de cette électrode pour éclairer la zone de peau sur laquelle est appliquée l'électrode, et il comporte d'autre part, dans un boîtier distinct, une unité électronique qui regroupe les différents moyens électriques en génération de courant de polarité déterminée, alternativement en mode pulsé ou en mode non pulsé (avantageusement toujours en courant continu, de même polarité, par distinction avec un courant alternatif), ainsi que des moyens programmés à paramètres variables pour la gestion des conditions de fonctionnement de l'appareil.

Plus précisément, l'appareil selon l'invention, dans ses modes de réalisation préférés, inclut des moyens à microprocesseurs qui sont programmés pour assurer automatiquement les commandes en distribution de courant électrique d'une part à ladite électrode, d'autre part aux différentes diodes, suivant un cycle de fonctionnement comportant les deux ou trois séquences du procédé défini ci-dessus.

En d'autres termes, l'appareil selon l'invention comporte des moyens de commande automatique pour commuter la production de courant électrique entre courant pulsé et courant non pulsé de même polarité à l'alimentation de l'électrode et pour commander en synchronisme l'allumage de diodes émettant de la lumière sous différentes couleurs dont chacune est associée à un type de courant à caractéristiques prédéfinies. On notera ici que si la commutation entre les couleurs d'illumination est synchronisée avec la commutation du type de courant appliqué à l'électrode, ceci ne veut pas nécessairement dire que les deux opérations s'effectuent strictement au même instant, puisqu'il peut, aussi bien, être prévu notamment que l'application d'un courant électrophorétique soit poursuivie alors que la couleur d'illumination est déjà passée de l'orange au bleu pour la dernière étape du cycle de fonctionnement.

Le plus souvent, l'appareil offrira plusieurs programmes de traitement prédéfinis au choix du praticien utilisant l'appareil. Les paramètres dont les valeurs pourront varier d'un programme à l'autre sont principalement les durées respectives de chacune des étapes de traitement. Leurs valeurs seront fixées dans chaque programme en correspondance avec le phototype, les différents types de peaux parmi les plus courants de la localisation de la peau à traiter, de la modification cosmétique à y apporter.

Par exemple, le procédé peut avoir une durée comprise entre 20 et 30 minutes.

Par exemple, la première séquence de soin de la peau peut avoir une durée permettant l'ouverture des pores de la peau. Avantageusement, cette durée peut être comprise entre 5 minutes et 30 minutes. La deuxième étape de soin de la peau peut avoir une durée permettant la migration des substances actives présentes sur la peau. Avantageusement, cette durée peut être comprise entre 1 minute et 10 minutes.

La troisième étape de soin de la peau peut avoir une durée peut avoir une durée permettant à la peau de se régénérer. Avantageusement, cette durée peut être comprise entre 0,5 minute et 10 minutes.

Les programmes prédéfinis peuvent aussi être paramétrés pour être utilisés chacun en association avec une composition de lotion parmi différentes lotions offertes au choix du praticien. L'un des paramètres est alors avantageusement la polarité du courant, venant en plus de la durée des différentes étapes dans les données fixées dans chaque programme. Quant à l'intensité du courant, elle reste de préférence réglable en permanence par le praticien, en fonction des sensations exprimées par le patient.

Dans les modes de mise en oeuvre préférées de l'invention (mais non limitatifs), les grandeurs qui sont ainsi paramétrables sont d'une part les durées respectives des différentes séquences de fonctionnement, d'autre part les caractéristiques d'intensité, polarité, fréquence de pulsation, du courant électrique appliqué dans chacune des séquences. En général, il n'est pas prévu d'instaurer une liberté de paramétrage pour les conditions d'illumination concomitantes, dès lors que l'on dispose par construction des couleurs rouge, orange et bleu, sauf le cas échéant pour ce qui concerne la puissance d'émission lumineuse dans chaque couleur. Le courant d'alimentation des sources lumineuses permet d'agir facilement sur leur puissance, d'où des valeurs qui peuvent être fixées différentes d'une couleur à l'autre et/ou de l'un à l'autre de plusieurs programmes enregistrés par avance dans l'unité électronique de pilotage de l'appareil.

La polarité du courant peut être positive ou négative, selon la polarité de la composition appliquée sur la peau. L'intensité du courant peut être comprise entre 1 mA et 10 mA, par exemple entre 2mA et 8 mA, ou encore entre 3 mA et 5mA pour toutes les séquences variable entre 0 et 10 mA

La fréquence de pulsation lors de la séquence d'électroporation peut être comprise entre 100 µs et 300 µs.

Du point de vue de sa construction mécanique, l'appareil suivant l'invention se caractérise principalement, dans ses modes de réalisation préférés, par le fait qu'au niveau de la tête d'application sur la peau, il comporte des diodes électroluminescentes d'illumination colorée de la peau qui sont réparties en couronne autour d'une électrode venant axialement au contact de la peau à traiter. Le plus avantageux est en général de prévoir plusieurs diodes pour chaque couleur, qui se succèdent intercalées en alternance dans les règles d'une symétrie de révolution autour de l'électrode. Ceci permet de fournir un éclairage homogène au voisinage immédiat de l'extrémité de l'électrode amenée en contact électrique avec la peau quelle que soit la couleur en cours.

Il est à noter que, selon un mode de réalisation préféré de l'invention, seule l'électrode de la tête de l'applicateur est destinée à entrer en contact avec la peau à traiter, les diodes n'entrant jamais en contact avec cette dernière et en étant protégées par une vitre isolante réalisée dans un matériau transparent au rayonnement visible, en général en verre minéral ou organique. De manière plus avantageuse encore, cette vitre est réalisée translucide.

Selon une autre caractéristique de l'invention, la tête de l'applicateur est conformée pour accueillir, en son extrémité destinée à être placée au contact de la peau à traiter, un quartz ou autre type de minéral à effet de lithothérapie quand il est amené en contact avec la peau en même temps que l'électrode. Cet élément est avantageusement disposé autour de l'électrode devant les diodes lumineuses dans la tête d'application sur la peau.

Avantageusement, le minéral peut augmenter ou amplifier l'effet du traitement. Le minéral peut par exemple améliorer la migration des substances actives présentes sur la peau, et/ou améliorer la décontraction ou relaxation de la peau. Sans que ce mécanisme d'action soit susceptible de lier la Demanderesse, il est possible que l'amplification soit liée à la piézo-électricité propre au minéral. La piézo-électricité du quartz peut par exemple augmenter la vascularisation de la peau, donc améliorer la migration des substances actives présentes sur la peau.

Comme la présence d'un quartz de lithothérapie rend la tête d'application relativement encombrante en sa partie terminale à appliquer sur la peau, il est en général souhaitable de disposer avec le même appareil d'une tête interchangeable non équipée d'un tel quartz, que l'on utilise alors pour le traitement de zones de peau difficiles d'accès, autour des paupières par exemple.

Quel que soit le mode de réalisation retenu, un ensemble de joints placés notamment entre l'électrode et la vitre translucide ou le quartz à effet de lithothérapie assurent l'étanchéité au regard de substances cosmétiques actives éventuellement déposées sur la peau préalablement à l'utilisation de l'appareil selon L'invention est définie dans les revendications attenantes.

D'autres caractéristiques et avantages de l'invention ressortiront des détails de réalisation pratiques d'un appareil particulier suivant l'invention, décrit ci-après en référence aux figures qui illustrent sa description, dans lesquelles :
- la figure 1 est une vue schématique de l'appareil selon l'invention montrant plus spécialement la constitution de l'ensemble électrique comportant son unité électronique de pilotage,
- la figure 2 est une vue schématique d'une première tête de l'appareil à appliquer sur la peau à traiter,
- la figure 3 est une vue schématique d'une autre tête d'applicateur du même appareil, interchangeable avec la première dans son utilisation,

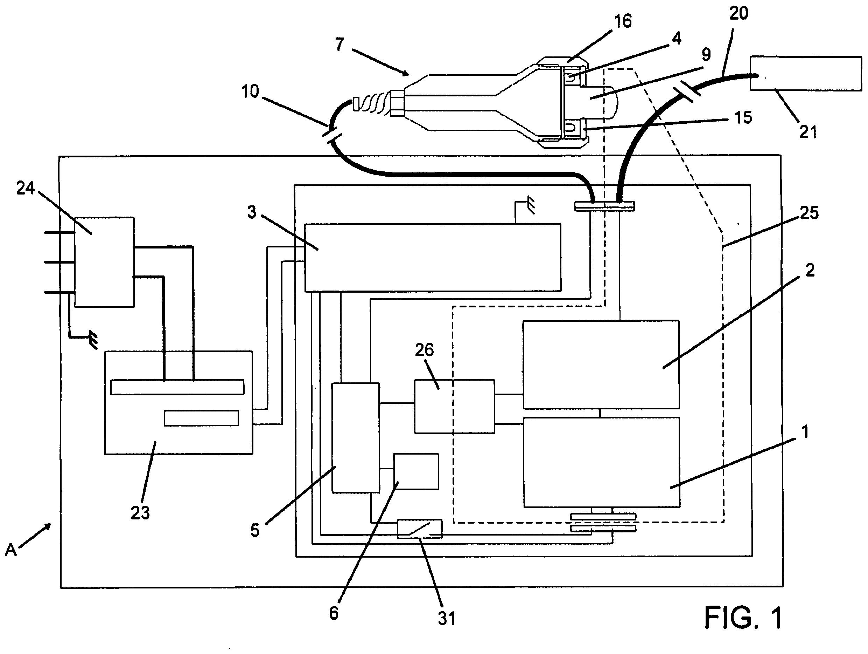

L'appareil suivant l'invention est ici décrit dans le cadre de son utilisation pour favoriser l'action de principes actifs contenus en mélange avec un véhicule approprié dans une composition sous forme de lotion de traitement cosmétique, que l'on dépose au préalable sur la zone de peau à traiter.
Les éléments essentiels de l'appareil se répartissent entre un boîtier électrique et électronique A et une tête de traitement 7 conformée pour être tenue à la main par le praticien. Cette tête de traitement présente une partie terminale à appliquer au contact de la peau en la déplaçant sur la surface de peau à traiter, à l'endroit des taches pigmentaires à traiter. C'est là que se situe, axialement, l'électrode 9 dont l'extrémité vient en contact avec la peau, là aussi que sont disposées les diodes émettrices de lumière colorée 4. Le circuit électrique de l'électrode se ferme, à travers le corps du patient, sur une électrode de référence opposée, schématisée en 21 sur la figure 1, qui est conformée pour être tenue par le patient en contact électrique avec la main. Le boîtier associé A contient un bloc d'alimentation électrique 3 à brancher sur une source extérieure par l'intermédiaire d'un filtre 24 et d'un transformateur 23. Le bloc 3 fournit l'énergie nécessaire à un générateur de courant d'intensité continue, ainsi qu'à un générateur impulsionnel 2. Le courant produit est dans les deux cas du courant sous basse tension, appliquant typiquement une différence de potentiel de 12 volts entre l'électrode de traitement 9 et l'électrode de référence 21, par l'intermédiaire des câbles électriques 10 et 20 respectivement.

L'intensité du courant délivré à l'électrode de traitement est variable sur une plage de valeurs déterminée, typiquement jusqu'à un maximum de 10 milliampères. Tout au long de chaque cycle de fonctionnement de l'appareil, l'intensité est réglable par action sur un bouton qui est accessible à l'utilisateur sur la façade du boîtier. Pour une efficacité optimale en électroporation, par opposition à l'efficacité recherchée en électrophorèse lors de l'application du courant non pulsé, le générateur de courant pulsé délivre des impulsions chacune d'une durée de 100 micro-secondes, avec une fréquence de répétition de quelques centaines de micro-secondes, typiquement fixée à 400 micro-secondes.

Le bloc 3 sert par ailleurs à l'alimentation électrique des diodes luminescentes, avec une puissance qui est prévue différente d'une couleur à l'autre dans le mode de réalisation préféré de l'appareil décrit ici. Cette puissance d'illumination de la peau en cours de traitement varie de plus forte à plus faible quand on passe d'une couleur plus chaude à une couleur plus froide. Elle est typiquement de 120 candelas pour la couleur rouge utilisée dans l'étape d'électroporation, de 56 candelas pour la couleur orange utilisée pendant l'étape d'électrophorèse, de 13 candelas pour la couleur bleu utilisée pendant l'étape finale de régénération de la peau.

En conformité avec l'invention, l'appareil décrit comporte une unité électronique 5 intégrant l'ensemble des moyens de pilotage des moyens d'alimentation électrique distribuant le courant aux diodes et à l'électrode. Ces moyens de pilotage sont disponibles sous la forme logicielle de programmes enregistrés dans une carte électronique appropriée, qui font intervenir différents paramètres auxquels il est assigné des valeurs différentes d'un programme à l'autre, en liaison avec les conditions de prescription de chaque programme.

En paramètres principaux, on trouve principalement la durée globale du cycle de traitement, qui peut varier typiquement entre 20 et 40 minutes, et qui est plus particulièrement de l'ordre de 20-25 à 30 minutes, ainsi que les durées respectives de chacune des étapes du cycle, avec notamment une durée de la séquence d'électroporation en général deux à trois fois plus longue par rapport aux durées de la séquence de traitement électrophorétique et de la séquence de régénération par chromothérapie. Une autre grandeur paramétrable de manière similaire, est représentée par la polarité du courant appliqué à l'électrode de traitement, une autre encore par la présence ou l'absence d'un quartz de lithothérapie dont on parlera plus loin.

Le praticien concepteur préparant les programmes enregistrés et l'unité de pilotage automatique fixera la valeur des paramètres de manière à adapter le traitement au mieux, par exemple au phototype, au type de peau du patient, à la localisation de la zone de peau à traiter, à son état du moment. Il pourra aussi, le cas échéant, tenir compte de la nature du produit appliqué sur la peau avant le traitement, de sa forme et de sa composition, en se référant à un code d'identification inscrit sur chaque ampoule contenant l'une des formulations de lotion à appliquer.

A titre d'exemple, un programme adapté pour le traitement de taches pigmentaires sur une peau claire et sèche, au niveau du front ou des pommettes, imposera un cycle de traitement de 24 minutes au total, comprenant l'application du courant pulsé d'électroporation sous exposition en lumière rouge pendant 15 minutes, puis l'application du courant d'électrophorèse sous exposition en lumière orange pendant 5 minutes, avant un traitement de chromothérapie sous couleur bleue pendant 4 minutes.

Les organes de commande de la commutation agissant sur le courant électrique appliqué à l'électrode agissent en synchronisme sur le courant d'alimentation des diodes d'illumination de la peau de manière à commander la commutation de l'allumage des différentes diodes pour passer d'une couleur à une autre. Ils commandent automatiquement le déroulement du cycle et des séquences de fonctionnement de l'appareil en tenant compte de la valeur donnée aux paramètres réglables pour chaque application particulière.

Un afficheur 6 (figure 1), ici constitué par un afficheur graphique à cristaux liquides rétro-éclairé, permet de visualiser les choix de l'utilisateur quant aux conditions de traitement qu'il souhaite appliquer (phototype, type de peau, localisation, lotion choisie, utilisation de l'électrode avec quartz ou de l'électrode sans quartz), ainsi que les références du programme de traitement que l'appareil détermine en conséquence, avec les valeurs des grandeurs paramétrables qui y auront été fixées automatiquement. Sur la figure 1 , on a également fait apparaître un interrupteur de mise en route 31 , ainsi qu'un bloc d'isolation 26 séparant le circuit d'alimentation des éléments optiques du circuit de production du courant de traitement vers l'électrode 9, lequel est isolé comme schématisé par le trait en tirets 25.

Préalablement à l'utilisation de l'appareil selon l'invention, la peau est avantageusement enduite de substances actives appropriées (par exemple sous forme de gels ou de crèmes) comportant des molécules spécifiques dont l'effet cosmétique est optimisé par les effets combinés des courants électriques et des rayons lumineux générés par les diodes luminescentes, ainsi que par l'effet éventuel de la présence d'un élément minéral tel qu'un quartz placé à l'extrémité de la tête de traitement. Pendant l'étape subséquente la chromothérapie sous couleur rouge combine ses effets avec ceux de l'électroporation par application d'un courant pulsé, pour assurer l'ouverture des pores de la peau. Ensuite, pendant l'étape d'électrophorèse en courant d'intensité continue sous lumière orange, certains programmes permettent d'augmenter l'intensité jusqu'à une valeur de l'ordre de 20 milliampères. L'exposition lumineuse prolonge l'effet d'ouverture des pores de l'étape d'électroporation, cependant que l'application du courant électrophorétique favorise la migration des substances actives présentes sur la peau. L'application du courant électrophorétique est en général poursuivie pendant l'étape finale qui utilise les diodes de couleur bleue pour provoquer une régénération des tissus favorable à un effet durable du traitement cosmétique réalisé.

Durant toutes ces étapes, courant électrique et rayonnement lumineux sont appliqués à la peau au moyen d'une tête de traitement 7 reliée aux générateurs de courant et aux moyens d'alimentation électrique des diodes par l'intermédiaire d'un raccord assurant les connexions électriques avec le boîtier A en bout d'un câble souple multibrins 10.

Dans la tête de traitement 7, de forme générale sensiblement cylindrique, l'électrode 9 est disposée dans l'axe longitudinal. Réalisée électriquement conductrice, elle est constituée d'un alliage métallique à base d'aluminium. L'électrode 9 est montée au centre d'une plaque 11 , disposée en travers de l'enveloppe isolante 14 de la tête de traitement. Cette plaque est elle-même constituée en un matériau électriquement isolant, par exemple à base de résine époxy. C'est sur elle que sont aussi montées les diodes électroluminescentes, alimentées par l'intermédiaire de pistes électriquement conductrices supportées par la même plaque.

Ainsi qu'il a été exposé précédemment, les diodes 4 sont arrangées alternées par couleur, de telle manière que l'éclairage fourni se répartisse de manière homogène en couronne autour de l'électrode 9, à proximité immédiate de celle-ci, ce pour chacune des couleurs considérées. Dans le cas particulier choisi pour illustrer au mieux l'invention, l'appareil comporte ainsi trois diodes de chacune des trois couleurs, réparties à intervalles angulaires égaux autour de l'électrode.

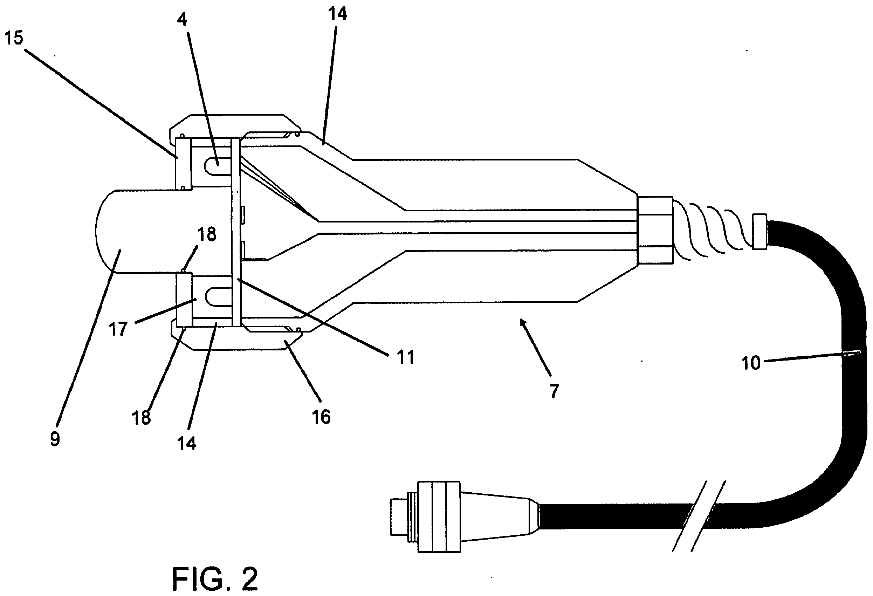

Selon le mode de réalisation de l'appareil illustré par la figure 2, les diodes sont montées en arrière d'une vitre protectrice en matériau translucide 15, qui s'interpose ainsi entre les diodes 4 et la peau contre laquelle la tête de traitement est placée. La vitre 15 a sensiblement la forme d'une rondelle de faible épaisseur, disposée autour de l'électrode 9 de telle manière que cette dernière se trouve en saillie pour entrer efficacement en contact avec la peau. Elle a essentiellement pour fonction de protéger les diodes 4 et la plaque 11 lors d'un nettoyage de l'appareil consécutif à une utilisation. Il est donc important de réaliser une bonne étanchéité afin d'isoler la plaque 11, porteuse des connexions électriques, ainsi que les diodes, du milieu extérieur.

Pour ce faire, une bague 16 est enfilée autour de la périphérie extérieure de l'élément transparent 15, et vissée sur la partie filetée de l'enveloppe isolante 14. La bague 16 solidarise avec celle-ci l'ensemble formé par la plaque 11, les diodes 4, la vitre 15 et l'électrode 9, en délimitant une cavité 17 dans laquelle sont accueillies les diodes 4. Un ensemble de joints toriques 18 réalisent l'étanchéité de la cavité 17 au regard du milieu environnant.

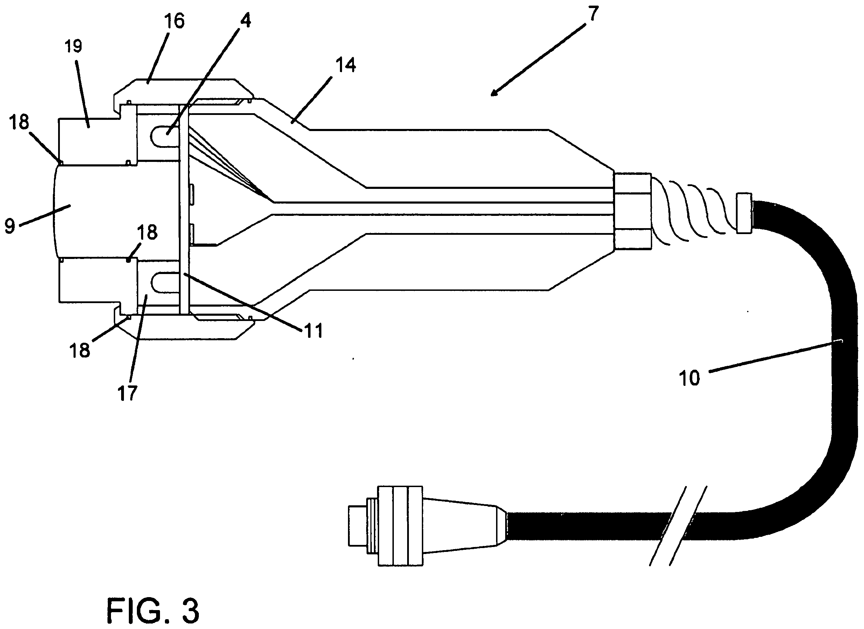

L'appareil décrit comporte une deuxième tête de traitement, représentée en figure 3, qui est prévue pour être utilisée en variante de la précédente, dont elle diffère par la présence d'un quartz à effet de lithothérapie quand il est amené au contact de la peau du patient.

Ce quartz 19 remplace la vitre 15, mais contrairement à celle-ci, il n'est pas en retrait de l'extrémité de l'électrode conductrice 9. Sa présence, combinée à celle des diodes de couleur et au courant délivré, augmente l'effet du traitement cosmétique réalisé, par sa mise en contact avec la peau à traiter concomitamment avec la mise en contact de l'électrode 9 avec cette dernière. Le quartz 19 est formé pour se placer autour de l'électrode 9, en couronne, avantageusement de manière à assurer un effet de lithothérapie se combinant avec les effets électriques d'électroporation et d'électrophorèse et avec les effets de chromothérapie des rayonnements colorés pour une action de soin cosmétique optimale.

On remarque sur la figure que l'électrode 9 est conformée pour se trouver en saillie légère par rapport à la face extérieure du quartz 19 pour assurer le contact électrique avec la peau, tout en présentant une extrémité aplatie afin de permettre un contact simultané de l'élément minéral avec la peau.

Le montage de la tête de l'applicateur est réalisé de manière similaire au montage illustré par la figure 2. Toutefois, le quartz 19 étant ici en contact étroit avec la surface extérieure de l'électrode 9 sur une partie importante de sa longueur, le joint torique 18 destiné à assurer l'étanchéité entre ces deux éléments est situé à proximité de l'extrémité de l'électrode 9 destinée à entrer en contact avec la peau. Ceci permet notamment d'éviter toute pénétration de traces de substances cosmétiques actives à l'interface entre l'électrode et le quartz, substances qui pourraient être à l'origine de pollutions et de corrosion de l'électrode. Il est également envisageable, comme illustré par la figure 3, de réaliser une double étanchéité à l'interface entre l'électrode 9 et le quartz 19, en plaçant un joint torique 18 au voisinage de chacune des faces de ce dernier.

Malgré ses avantages, la tête de traitement à quartz de lithothérapie, ne prive pas la tête sans quartz de son utilité. L'une et l'autre sont à utiliser suivant l'accessibilité de la zone de peau à traiter. Des programmes de pilotage différents sont associés à chacune des deux têtes de traitement. Par exemple, l'utilisateur changera de programme en même temps qu'il passera d'une application sur le front à l'aide de la tête à quartz au traitement des paupières ou du tour des yeux au moyen de la tête sans quartz. D'autres avantages pourront encore apparaître à l'homme du métier à la lecture des exemples de mise en oeuvre ci-dessous, illustrés par les figures annexées, donnés à titre illustratif.

### EXEMPLE 1 : MISE EN OEUVRE DE L'APPAREIL DE SOIN COMPRENANT UNE TETE MUNIE D'UN MINÉRAL

### 1) Caractéristiques de l'appareil

On utilise un appareil de soin qui comprend : - des moyens (1 ,2) de production d'un courant électrique de polarité déterminée, entre une électrode (9) à appliquer sur la peau et une électrode opposée à porter à un potentiel de référence,
- des moyens d'illumination (4) producteurs d'un rayonnement lumineux coloré au voisinage de l'électrode, - en bout d'un boîtier isolant à tenir à la main par un praticien utilisateur de l'appareil, un morceau de quartz de forme arrondie d'environ 4,1 mm de diamètre à effet de lithothérapie venant en contact avec la peau en même temps que l'électrode autour de celle-ci,
- une unité électronique programmée pour commander automatiquement la commutation entre la production d'un courant pulsé d'électroporation en première étape d'un cycle de fonctionnement et celle d'un courant non pulsé d'électrophorèse en seconde étape dudit cycle, en synchronisme avec la commutation de la production dudit rayonnement coloré.

Les moyens d'illumination et les moyens producteurs de courant sont intégrés dans une tête d'application, en bout d'un boîtier isolant.

### 2) Soin d'un individu avec l'appareil

Une lotion d'acide salicylique est appliquée sur chaque pommette, joue et tempe de chaque côté du visage de la peau du visage d'un individu, marquée par des taches pigmentaires.

L'appareil est alors appliqué sur une pommette, une joue et une tempe d'un côté du visage à la surface de laquelle est posée la lotion de soin.

La peau est alors soumise, via l'appareil, à une première séquence de soin, par application d'un courant pulsé simultanément à l'exposition de la peau à une lumière rouge, pendant 12 minutes. La fréquence des pulsations est de 100 µs en temps de travail et de 300 µs en temps de repos. L'intensité du courant est de 10 mA. L'intensité lumineuse est de120 candelas.

Puis, la peau est soumise, via l'appareil, à une deuxième séquence de soin, par application d'un courant continu simultanément à l'exposition de la peau à une lumière orange, pendant environ 8 minutes. L'intensité du courant est variable de 0 à 10 mA. L'intensité lumineuse est de 56 candelas.

La peau est enfin soumise à une troisième séquence de soin, par application d'un courant continu simultanément à l'exposition de la peau à une lumière bleue, pendant environ 4 minutes. L'intensité du courant est de 0 à 10 mA. L'intensité lumineuse est de 13 candelas. Un contrôle est réalisé sur le même individu, sur le côté du visage n'ayant pas reçu le traitement par l'appareil.

Les effets du traitement au moyen de la crème et de l'appareil d'une part, et au moyen de la crème seule d'autre part, sont observés visuellement et comparés, éventuellement après chaque séance de traitement. Par ailleurs, une observation visuelle est réalisée sur le côté du visage ayant bénéficié du traitement avec l'appareil, avant application de l'appareil et après l'application du traitement avec l'appareil. On observe visuellement une réduction de la couleur (imprégnation) des taches pigmentaires d'environ 20%.

### EXEMPLE 2 : MISE EN OEUVRE DE L'APPAREIL DE SOIN POUR LES ZONES D'ACCES DIFFICILE

### 1) Caractéristiques de l'appareil

On utilise un appareil de soin qui comprend :
- des moyens (1 ,2) de production d'un courant électrique de polarité déterminée, entre une électrode (9) à appliquer sur la peau et une électrode opposée à porter à un potentiel de référence, - des moyens d'illumination (4) producteurs d'un rayonnement lumineux coloré au voisinage de l'électrode, et
- une unité électronique programmée pour commander automatiquement la commutation entre la production d'un courant pulsé d'électroporation en première étape d'un cycle de fonctionnement et celle d'un courant non pulsé d'électrophorèse en seconde étape dudit cycle, en synchronisme avec la commutation de la production dudit rayonnement coloré.

Les moyens d'illumination et les moyens producteurs de courant sont intégrés dans une tête d'application, en bout d'un boîtier isolant.

### 2) Soin d'un individu avec l'appareil

Une lotion d'acide glycolique de soin esthétique est appliquée sur chaque cerne de la peau du visage d'un individu.

L'appareil est alors appliqué sur une des deux cernes. La peau est alors soumise, via l'appareil, à une première séquence de soin, par application d'un courant pulsé simultanément à l'exposition de la peau à une lumière rouge, pendant 12 minutes. La fréquence des pulsations est de 100µs en temps de travail et 300µs en temps de repos. L'intensité du courant est comprise entre 0 mA et 10 mA. L'intensité lumineuse est de 120 candelas. Puis, la peau est soumise, via l'appareil, à une deuxième séquence de soin, par application d'un courant continu simultanément à l'exposition de la peau à une lumière orange, pendant environ 8 minutes. L'intensité du courant est de 10mA. L'intensité lumineuse est de 56 candelas.

La peau est enfin soumise à une troisième séquence de soin, par application d'un courant continu simultanément à l'exposition de la peau à une lumière bleue, pendant environ 4 minutes. L'intensité du courant est de 10mA. L'intensité lumineuse est de 13 candelas.

Un contrôle est réalisé sur le même individu, sur le cerne n'ayant pas reçu le traitement par l'appareil. Les effets du traitement au moyen de la crème et de l'appareil d'une part, et au moyen de la crème seule d'autre part, sont observés visuellement et comparés, éventuellement après chaque séance de traitement.

Par ailleurs, une observation visuelle est réalisée sur le cerne ayant bénéficié du traitement avec l'appareil, avant application de l'appareil et après l'application du traitement avec l'appareil. On observe visuellement une réduction de la couleur (imprégnation) des taches pigmentaires d'environ 20% à 30%.

## Revendications

1. Procédé de soin esthétique de la peau, à l'exclusion de tout traitement thérapeutique, **caractérisé en ce qu'**il consiste à soumettre la peau à l'activation d'un quartz piézo-électrique à effet de lithothérapie dans une tête d'application sur la peau et à un cycle de séquences successives comportant, dans cet ordre, au moins une première séquence de soin de la peau, dite d'électroporation, comportant l'application sur la peau d'un courant pulsé d'électroporation en combinaison avec l'exposition simultanée de la peau en cours de soin à de la lumière dont la longueur d'ondes est comprise entre 692 nm et 800 nm, et une seconde séquence de soin de la peau, dite d'électrophorèse, comportant l'application d'un courant continu d'électrophorèse en combinaison avec l'exposition simultanée de la peau en cours de soin à de la lumière de longueur d'ondes inférieure à 692 nm.

2. Procédé selon la revendication 1, dans lequel ladite première séquence d'électroporation s'effectue sous exposition à une lumière de couleur rouge et ladite seconde séquence d'électrophorèse s'effectue sous exposition à de la lumière de couleur orange.

3. Procédé selon l'une des revendications 1 ou 2, comportant une troisième séquence de soin de la peau, dite de régénération, consistant en une étape de chromothérapie sous exposition à de la lumière de couleur froide, dont la longueur d'onde est comprise entre 330 nm et 560 nm, notamment de couleur bleue.

4. Procédé selon la revendication 3, dans lequel l'application d'un courant à action électrophorétique est poursuivie pendant ladite séquence de régénération de la peau.

5. Procédé selon l'une des revendications 1 à 4, dans lequel, préalablement audit cycle d'application d'un courant et d'exposition simultanée à de la lumière colorée, on dépose sur la peau une composition cosmétique, avantageusement sous forme de lotion.

6. Appareil de traitement de la peau, **caractérisé en ce qu'**il comporte:
- des moyens d'activation d'un quartz piézo-électrique à effet de lithothérapie positionné dans une tête d'application au contact de la peau,
- des moyens (1, 2) de production d'un courant électrique de polarité déterminée, alternativement en mode continu ou en mode pulsé, entre une électrode (9) à appliquer sur la peau et une électrode opposée à porter à un potentiel de référence,
- des moyens d'illumination (4) producteurs d'un rayonnement lumineux coloré, alternativement d'au moins deux couleurs différentes, en direction de la peau au voisinage de ladite électrode, et
- une unité électronique programmée pour commander automatiquement, pendant l'activation continue du quartz à effet de lithothérapie :
- la commutation entre :
- la production d'un courant pulsé d'électroporation en première étape d'un cycle de fonctionnement, par les moyens de production d'un courant électrique, et
- la production d'un courant non pulsé d'électrophorèse en seconde étape dudit cycle, par les moyens de production d'un courant électrique ; en synchronisme avec
- la commutation entre
- la production dudit rayonnement coloré dont la longueur d'onde est comprise entre 692nm et 800nm, par les moyens d'illumination, et
- la production dudit rayonnement coloré d'une couleur de longueur d'onde inférieure à 692 nm, par les moyens d'illumination.

7. Appareil de traitement de la peau selon la revendication 6, dans lequel ladite unité électronique est programmée pour commander en outre automatiquement la production par lesdits moyens d'illumination d'un rayonnement coloré d'exposition de la peau à une couleur froide, dont la longueur d'onde est comprise entre 330 nm et 560 nm, telle que le bleu, pendant une troisième étape dudit cycle de fonctionnement, au cours de laquelle l'application d'un courant continu d'électrophorèse est avantageusement poursuivie.

8. Appareil de traitement de la peau selon l'une des revendications 6 ou 7, dans lequel lesdits moyens d'illumination comportent des diodes électro-luminescentes (4) émettrices de faisceaux lumineux de différentes couleurs qui sont réparties en couronne en arrière d'une vitre protectrice (15) disposée en retrait d'une extrémité de ladite électrode à appliquer en contact avec la peau à traiter, à l'intérieur d'un boîtier isolant à tenir à la main par un praticien utilisateur de l'appareil.

9. Appareil de traitement de la peau selon la revendication 8, dans lequel lesdites diodes sont émettrices de rayonnement lumineux de trois couleurs différentes, respectivement dans le rouge, l'orange et le bleu, qui se succèdent en alternance autour de ladite électrode.

10. Appareil de traitement de la peau selon l'une des revendications 6 à 9, dans lequel ladite électrode (9) et lesdits moyens d'illumination (4) sont intégrés dans une tête d'application, en bout d'un boîtier isolant à tenir à la main par un praticien utilisateur de l'appareil, avec un quartz à effet de lithothérapie venant en contact avec la peau en même temps que l'électrode autour de celle-ci.

## Patentansprüche

1. Kosmetisches Hautpflegeverfahren unter Ausschluss jeder weiteren therapeutischen Behandlung, **dadurch gekennzeichnet, dass** es darin besteht, die Haut der Aktivierung eines piezoelektrischen, lithotherapeutisch wirkenden Quarz in einem Applikationskopf auf der Haut und einem Zyklus aufeinander folgender Sequenzen zu unterziehen, umfassend, in der angegebenen Reihenfolge, mindestens eine erste Hautpflegesequenz, die so genannte Elektroporation, welche die Anwendung eines pulsierenden Elektroporationsstroms auf der Haut in Kombination mit der gleichzeitigen Exposition der behandelten Haut gegenüber Licht mit einer Wellenlänge zwischen 692 nm und 800 nm beinhaltet, und eine zweite Hautpflegesequenz, die so genannte Elektrophorese, welche die Anwendung eines kontinuierlichen Elektrophoresestroms in Kombination mit der gleichzeitigen Exposition der behandelten Haut gegenüber Licht mit einer Wellenlänge unter 692 nm beinhaltet.

2. Verfahren nach Anspruch 1. bei dem die besagte erste Elektroporationssequenz unter Einwirkung von rotem Licht und die besagte zweite Elektrophoresesequenz unter Einwirkung von orangenem Licht durchgeführt wird.

3. Verfahren nach einem der Ansprüche 1 oder 2, das eine dritte Hautpflegesequenz umfasst, die so genannte Regenerationssequenz, die aus einer Chromotherapiestufe unter Einwirkung von Kaltlicht, mit einer Wellenlänge zwischen 330 nm und 560 nm, vor allem Blaulicht besteht.

4. Verfahren nach Anspruch 3, bei dem ein elektrophoretisch wirkender Strom während der genannten Hautregenerationssequenz angewendet wird.

5. Verfahren nach einem der Ansprüche 1 bis 4, bei dem vor dem besagten Zyklus der Stromanwendung und der gleichzeitigen Einwirkung von farbigem Licht eine kosmetische Zusammensetzung, vorzugsweise in Form einer Lotion, auf die Haut aufgetragen wird.

6. Gerät zur Hautbehandlung, **dadurch gekennzeichnet, dass** es folgendes umfasst:
- Mittel zur Aktivierung eines piezoelektrischen, lithotherapeutisch wirkenden Quarz, der in einem Applikationskopf positioniert wird, der mit der Haut in Kontakt ist.
- Mittel (1,2) zur Erzeugung eines elektrischen Stroms mit bestimmter Polarität, abwechselnd im kontinuierlichen oder im gepulsten Modus, zwischen einer Elektrode (9) zum Aufbringen auf die Haut und einer Gegenelektrode zum Anlegen auf einem Bezugspotential.
- Beleuchtungsmittel (4) zur Erzeugung einer farbigen Lichteinstrahlung, abwechselnd in mindestens zwei verschiedenen Farben, auf die Haut in der Umgebung der besagten Elektrode und
- eine elektronische Einheit, die während der kontinuierlichen Aktivierung des lithotherapeutisch wirkenden Quarz zur automatischen Steuerung programmiert ist
- der Umschaltung zwischen:
- der Erzeugung eines gepulsten Elektroporationsstroms in der ersten Stufe eines Betriebszyklus durch Mittel zur Erzeugung eines elektrischen Stroms und
- der Erzeugung eines nicht gepulsten Elektrophoresestroms in der zweiten Stufe des besagten Zyklus durch Mittel zur Erzeugung eines elektrischen Stroms;
synchron mit
- der Umschaltung zwischen
- der Erzeugung der besagten farbigen Strahlung mit einer Wellenlänge zwischen 692 nm und 800 nm durch die Beleuchtungsmittel und
- der Erzeugung der besagten farbigen Strahlung mit einer Wellenlänge unter 692 nm durch die Beleuchtungsmittel.

7. Gerät zur Hautbehandlung nach Anspruch 6, bei dem die genannte elektronische Einheit programmiert ist, außerdem die Erzeugung einer farbigen Strahlung durch die besagten Beleuchtungsmittel zur Bestrahlung der Haut mit Kaltlicht, mit einer Wellenlänge zwischen 330 nm und 560 nm, wie zum Beispiel Blaulicht, in einer dritten Stufe des besagten Betriebszyklus, während der vorzugsweise ein kontinuierlicher Elektrophoresestrom angewendet wird, automatisch zu steuern.

8. Gerät zur Hautbehandlung nach einem der Ansprüche 6 oder 7, bei dem die genannten Beleuchtungsmittel Leuchtdioden (4) aufweisen, die Strahlenbündel in verschiedenen Farben abgeben, die als Kranz hinter einem Schutzglas (15) verteilt sind, das hinter einem Ende der genannten Elektrode, die direkt auf der zu behandelnden Haut angelegt wird, in einem isolierenden Gehäuse angeordnet ist, das von einem Arzt zur Bedienung des Geräts in der Hand gehalten wird.

9. Gerät zur Hautbehandlung nach Anspruch 8, bei dem die genannten Dioden eine Lichtstrahlung in drei verschiedenen Farben aussenden, jeweils in rot, orange und blau, die um die genannte Elektrode abwechselnd aufeinander folgen.

10. Gerät zur Hautbehandlung nach einem der Ansprüche 6 bis 9, bei dem die genannte Elektrode (9) und die genannten Beleuchtungsmittel (4) in einem Applikationskopf, am Ende eines isolierenden Gehäuses integriert sind, das von einem Arzt, der das Gerät bedient, in der Hand gehalten wird, mit einem lithotherapeutisch wirkenden Quarz, der zur gleichen Zeit mit der Haut in Kontakt kommt wie die um die Haut angelegte Elektrode.

## Claims

1. Method of esthetic skin care, excluding any therapeutic treatment, **characterized in that** it comprises subjecting the skin to the activation of a piezoelectric quartz with a lithotherapy-effect
in an applicator head on the skin,
a cycle of successive sequences comprising, in this order, at least a first sequence of skin care, known as electroporation, comprising the application of a pulsed electroporation current on the skin in combination with simultaneous exposure of the skin during treatment to light with a wavelength between 692 nm and 800 nm, and a second skin care sequence, known as electrophoresis, comprising the application of a continuous electrophoresis current in combination with simultaneous exposure of the skin during treatment to light with a wavelength lower than 692 nm.

2. Method according to claim 1, wherein said first sequence of electroporation is carried out under exposure to a light of a red color, and said second sequence of electrophoresis is carried out under exposure to light of an orange color.

3. Method according to one of claims 1 or 2, comprising a third skin care sequence, known as regeneration, consisting of a chromotherapy step under exposure to a light of a cold color, with a wavelength between 330nm and 560nm, in particular blue.

4. Method according to claim 3, wherein the application of a current with an electrophoretic action is carried out during said skin regeneration sequence.

5. Method according to one of claims 1 to 4, wherein, prior to said cycle of applying a current and simultaneous exposure to colored light, a cosmetic compound is applied, advantageously in the form of a lotion.

6. Skin treatment apparatus, **characterized in that** it comprises:
- means for activating a piezoelectric quartz with a lithotherapy-effect placed in an applicator head in contact with the skin;
- means (1, 2) for producing an electric current with determined polarity, alternately in continuous mode or in pulsed mode, between an electrode (9) to be applied on the skin and an opposite electrode to be brought to a reference potential;
- illumination means (4) producing a colored light radiation, of at least two different colors alternately, in the direction of the skin near said electrode; and
- an electronics unit programmed to automatically control during the continuous activation of the quartz with a lithotherapy-effect:
- the switch between:
- production of a pulsed electroporation current in a first step of a cycle of operation, by the means for producing an electric current; and
- production of a non-pulsed electrophoresis current in a second step of said cycle, by the means for producing an electric current;
synchronized with
- the switch between
- production of said colored radiation with a wavelength between 692 nm and 800 nm, by the illumination means; and
- production of said colored radiation with a wavelength lower than 692 nm, by the illumination means.

7. Skin treatment apparatus according to claim 6, wherein said electronics unit is programmed to also automatically control the production by said illumination means of a colored radiation for exposing the skin to a cold color, , with a wavelength between 330 nm and 560 nm, such as blue, during a third step of said cycle of operation, during which the application of a continuous electrophoresis current is advantageously carried out.

8. Skin treatment apparatus according to one of claims 6 or 7, wherein said illumination means comprise light-emitting diodes (4) emitting light beams of different colors distributed in a ring behind a protective sheet (15) positioned some distance from the end of said electrode to be applied in contact with the skin to be treated, inside an insulating housing to be held in the hand of a practitioner using the apparatus.

9. Skin treatment apparatus according to claim 8, wherein said diodes emit three different colors of light radiation, respectively red, orange and blue, which alternate around said electrode.

10. Skin treatment apparatus according to one of claims 6 to 9, wherein said electrode (9) and said illumination means (4) are incorporated in an applicator head, at the end of an insulating housing to be held in the hand of a practitioner using the apparatus, with a lithotherapy-effect quartz coming into contact with the skin at the same time as the electrode around it.
